(19) Europäisches Patentamt
European Patent Office
Office européen des brevets

(11) Veröffentlichungsnummer: **0 442 326 B1**

# (12) EUROPÄISCHE PATENTSCHRIFT

(45) Veröffentlichungstag der Patentschrift: **21.12.94**

(51) Int. Cl.$^5$: **C08F 220/12**, C08F 2/44, A61K 6/083

(21) Anmeldenummer: **91101260.7**

(22) Anmeldetag: **31.01.91**

## (54) Füllstoffhaltige, quellbare Perlpolymerisate.

(30) Priorität: **15.02.90 DE 4004678**

(43) Veröffentlichungstag der Anmeldung:
**21.08.91 Patentblatt 91/34**

(45) Bekanntmachung des Hinweises auf die Patenterteilung:
**21.12.94 Patentblatt 94/51**

(84) Benannte Vertragsstaaten:
**AT BE CH DE DK ES FR GB GR IT LI LU NL SE**

(56) Entgegenhaltungen:
**DE-A- 3 201 109**

(73) Patentinhaber: **BAYER AG**

**D-51368 Leverkusen (DE)**

(72) Erfinder: **Podszun, Wolfgang, Dr.**
**Roggendorfstrasse 55**
**W-5000 Köln 80 (DE)**
Erfinder: **Winkel, Jens, Dr.**
**Letterhausstrasse 1**
**W-5000 Köln 71 (DE)**
Erfinder: **Müller, Michael, Dr.**
**Richard-Zanders-Strasse 34**
**W-5060 Bergisch-Gladbach 2 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft füllstoffhaltige, quellbare Perlpolymerisate, ihre Herstellung und ihre Verwendung in polymerisierbaren Dentalmassen. Es ist z.B. aus der US 4 396 377 bekannt, vernetzte quellbare Perlpolymerisate auf Basis von Methacrylsäureestern in Dentalwerkstoffen, insbesondere zur Herstellung von künstlichen Zähnen und Prothesen einzusetzen. Da diese Perpolymerisate nicht durch anorganische Füllstoffe verstärkt sind, zeigen die daraus hergestellten Werkstoffe für einige Einsatzgebiete, wie z.B. die Verwendung als Füllungsmaterial im Seitenzahnbereich zu geringe mechanische Festigkeiten.

Vernetzte füllstoffhaltige Perlpolymerisate werden in DE-A-284 936 und DE-A-3 201 109 beschrieben. Diese Perlpolymerisate eignen sich in hervorragender Weise als Komponenten von Zahnfüllungsmaterialien. Durch Abmischen mit monomeren Bindern werden pastöse Zubereitungen erhalten, die gute Verarbeitungseigenschalten aufweisen und durch Härtung in Werkstoffe mit hoher Transparenz und hoher mechanischer Festigkeit überführt werden können (DE-A-2 850 917). Die bekannten füllstoffhaltigen Perlpolymerisate werden von den Monomeren der polymerisierbaren Dentalwerkstoffe nicht oder nur sehr wenig angequollen.

Für die Anwendung als Zahnfüllungsmaterial im Seitenzahnbereich sind polyzmerisierbare, pastöse Zubereitungen mit fester Konsistenz besonders vorteilhaft, da diese nach der Stopftechnik verarbeitet werden können und gut modellierbar sind.

Polymerisierbare Zubereitungen mit fester Konsistenz sind in der Technik nur schwer herstellbar, da infolge der hohen Mischungswärme, der geringen Wärmeleitfähigkeit und der geringen Sauerstoffdiffusionsgeschwindigkeit eine unerwünschte, vorzeitige Polymerisation der Zubereitung während des Herstellungsprozesses eintreten kann.

Das vorzeitige Polymerisieren kann zwar in gewissen Grenzen durch die technischen Randbedingungen, wie Ansatzgröße, Geometrie des Mischaggregates, Mischleistung und Temperaturführung beeinflußt werden, eine grundsätzliche Lösung des Problems ist jedoch auf diesem Wege nicht möglich.

Eine der vorliegenden Erfindung zugrundeliegende Aufgabe besteht daher darin, eine Komponente für Dentalwerkstoffe, insbesondere für Zahnfüllungsmaterialien bereitzustellen, die die Herstellung von Zubereitungen mit besonders fester Konsistenz ermöglicht bzw. erleichtert. Eine weitere Aufgabe besteht darin, eine Komponente für Dentalwerkstoffe bereitzustellen, die sowohl die Verarbeitungseigenschaften als auch die Endeigenschaften des Werkstoffes in vorteilhafter Weise beeinflußt.

Diese Aufgaben werden durch den Einsatz vernetzter Perlpolymerisate, die dadurch gekennzeichnet sind, daß sie einen mittleren Teilchendurchmesser von 2 bis 100 $\mu$m, einen Gehalt an anorganischem Füllstoff von 10 bis 75 Gew.-% und einen Quellungsgrad von 50 bis 2.000 %, gemessen in THF, aufweisen, gelöst.

Die vernetzten Perlpolymerisate sind vorzugsweise aus den folgenden Komponenten aufgebaut:

| | | |
|---|---|---|
| A: | 10 bis 78 Gew.-% | $C_1$-$C_2$-Alkyl-(meth)acrylat |
| B: | 5 bis 50 Gew.-% | $C_3$-$C_{12}$-Alkyl-(meth)acrylat |
| C: | 1 bis 30 Gew.-% | Vernetzer |
| D: | 10 bis 75 Gew.-% | anorganischer Füllstoff |
| E: | 0 bis 30 Gew.-% | weitere ethylenisch ungesättigte Monomere |

Mit der Bezeichnung (Meth)acrylat ist sowohl das Acrylat als auch das Methacrylat gemeint, $C_1$-$C_2$-Alkyl(meth)acrylate (Komponente A) sind Methylacrylat, Methylmethacrylat, Ethylacrylat und Ethylmethacrylat. Bevorzugt ist Methylmethacrylat.

$C_3$-$C_{12}$-Alkyl(meth)acrylate (Komponente B) sind aliphatische, verzweigt aliphatische und cycloaliphatische Ester der Acryl- und Methacrylsäure. Beispielhaft seien genannt: n-Propylacrylat, i-Propylacrylat, i-Propylmethacrylat, n-Butylacrylat, n-Hexylacrylat, n-Hexylmethacrylat, 2-Ethylhexylacrylat, n-Decylmethacrylat, n-Decylacrylat, n-Dodecylmethacrylat sowie Cyclohexylmethacrylat.

Geeignet sind auch Alkyl(meth)acrylate, bei denen die Kohlenstoffkette durch Sauerstoff unterbrochen ist wie 2-Methoxyethylmethacrylat, 3-Methoxybutylmethacrylat und Ethyltriglykolmethacrylat.

Vernetzer (Komponente C) sind (Meth)acrylate mit 2 oder mehr, bevorzugt 2 bis 4, polymerisierbaren Doppelbindungen im Molekül, wie z.B.:

Ethylenglykoldimethacrylat, Diethylenglykoldimethacrylat, Triethylenglykoldimethacrylat, Glycerindimethacrylat, Glycerintrimethacrylat, Trimethylolpropantrimethacrylat, Trimethylolpropantriacrylat, Pentaerythritdimethacrylat, Pentaerythrittrimethacrylat,Pentaerythrittetramethacrylat, Derivate des Bisphenol A, wie Bisphenol-A-dimethacrylat und Bisphenol-A-diglykoldimethacrylat, Bisphenol-A-diglycidyldimethacrylat, Urethanmethacrylate, die durch Umsetzung von Diisocyanaten und Hydroxyalkylmethacrylaten hergestellt werden

können, wie

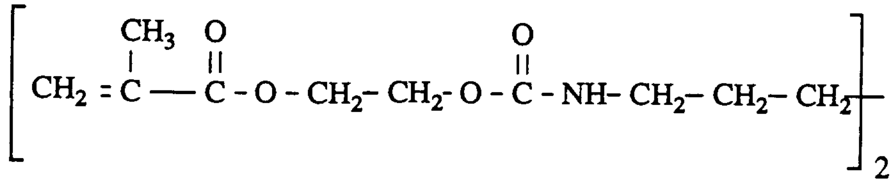

und Umsetzungsprodukte aus Polyolen, Diisocyanaten und Hydroxyalkylmethacrylaten (DE-A 37 03 080, DE-A 37 03 130 und DE-A 37 03 120).

Als anorganische Füllstoffe (Komponente D) sind farblose anorganische Substanzen, wie Quarz, Kristobalit, Quarzglas, hochdisperse Kieselsäure, Silikate, Aluminosilikate, Silikatgläser und Glaskeramiken geeignet. Die Füllstoffe weisen im allgemeinen einen mittleren Teilchendurchmesser von 0,01 bis 10 µm, vorzugsweise 0,02 bis 5 µm, auf. Bevorzugte Füllstoffe sind solche auf $SiO_2$-Basis, die eine hohe spezifische Oberfläche, beispielsweise 50 bis 400 $m^2/g$, gemessen nach der BET-Methode, aufweisen.

Zur Erzielung hoher Füllgrade und eines günstigen Eigenschaftsspektrums können Füllstoffe mit bimodaler Verteilung oder Füllstoffkombination eingesetzt werden.

Die anorganischen Füllstoffe gelangen vorzugsweise in mit Haftvermittlern behandelter Form zum Einsatz. Als Haftvermittler eignen sich beispielweise Silan- und Titanatverbindungen, wie Trimethylchlorsilan, Hexamethyldisiloxan, 3-Aminopropyltrimethoxysilan, Butyltitanat und Isopropyltitanat. Besonders gut geeignet sind Haftvermittler mit polymerisierbaren Gruppen, wie Vinyltrimethylsiloxan, Allyltrimethoxysilan und γ-Methacryloyloxypropyltrimethoxysilan.

Die Menge von Haftvermittlern beträgt 0,1 bis 10 Gew.-%, vorzugsweise 0,5 bis 5 Gew.-%, bezogen auf den anorganischen Füllstoff.

Die vernetzten Perlpolymerisate können als Komponente E bis zu 30 %, vorzugsweise bis zu 20 %, weitere ethylenisch ungesättigte Monomere, die mit den Komponenten A-C copolymerisierbar sind, enthalten. Als Beispiele seien genannt: Stryol, α-Methylstyrol, Vinyltoluol, Acrylnitril, Methacrylnitril, Vinylacetat und Vinylpropionat.

Das Verfahren zur Herstellung der erfindungsgemäßen Perlpolymerisate ist ebenfalls Gegenstand der vorliegenden Erfindung. Dieses Verfahren ist dadurch gekennzeichnet, daß man eine Mischung I aus

A: 10 bis 78 Gew.-Teilen $C_1$-$C_2$-Alkyl-(meth)acrylat

B: 5 bis 50 Gew.-Teilen $C_3$-$C_{12}$-Alkyl-(meth)acrylat

C: 1 bis 30 Gew.-Teilen Vernetzer

D: 10 bis 75 Gew.-Teilen anorganischen Füllstoff

E: 0 bis 30 Gew.-Teilen weiteren ethylenisch ungesättigten Monomeren

und

F: 50 bis 1.000 Gew.-Teilen nichtwäßrigen Verdünnungsmittel in einer wäßrigen Polymerisatlösung II dispergiert und unter Anwendung eines Initiators nach der Verfahrensweise der Suspensionspolymerisation polymerisiert.

Als nichtwäßrige Verdünnungsmittel (Komponente F) sind Flüssigkeiten geeignet, die die Monomeren gut und unvernetzte Polymere, die aus den Komponenten A, B und E aufgebaut sind, zumindest teilweise lösen. Die Lösungseigenschaften können durch den sogenannten Löslichkeitsparameter definiert werden (H.G. Elias, Makromoleküle, S. 192-196 (1981)). Für das erfindungsgemäße Verfahren verwendet man Verdünnungsmittel mit einem Parameter von 8 bis 15 [$cal^{0,5}cm^{-1,5}$], bevorzugt von 8,5 bis 13 [$cal^{0,5}cm^{-1,5}$].

Beispielsweise seien die folgenden Verdünnungsmittel genannt. Amylacetat, Butylacetat, Propylacetat, Ethylacetat, Ethylpropionat, Butanon-2, Pentanol, Hexanol, Heptanol, Octanol, 1,2-Dichlorethan, Tetrachlorethan, Dichlormethan, Trichlormethan, Toluol, Chlorbenzol. Besonders gut geeignet ist Toluol.

Die Menge des eingesetzten Verdünnungsmittels beeinflußt neben der Menge an Vernetzer ganz wesentlich den Quellungsgrad. Im allgemeinen wird das Verdünnungsmittel in Mengen von 50 bis 1.000 Gew.-%, vorzugsweise 100 bis 500 Gew.-%, bezogen auf die Summe der Komponenten A-E, eingesetzt.

Im ersten Schritt des erfindungsgemäßen Verfahrens werden die Komponenten A-F intensiv gemischt. Um eine gute Dispergierung des Füllstoffes zu erzielen, werden vorzugsweise Mischaggregate verwendet, die hohe Scherkräfte erzeugen, z.B. Schnellrührer, Rotorstatormischer oder Kugelmühlen.

In einer besonderen Ausführungsform des Verfahrens wird die Oberflächenbehandlung des Füllstoffs mit den oben genannten Haftvermittlern in der erhaltenen Dispersion durchgeführt. Hierbei werden selbstverständlich die für die Oberflächenbehandlung notwendigen Katalysatoren eingesetzt. Für die Oberflächenbehandlung mit Alkylsilanverbindungen sind z.B. Amine, wie Dicyclohexylamin gut geeignete Katalysatoren.

Als Initiator zur Einleitung der Polymerisation eignen sich an sich bekannte Radikalbildner, wie aliphatische Azodicarbonsäurederivate wie Azobisisobuttersäurenitril oder Azodicarbonsäureester, Peroxide wie Lauroylperoxid, Succinylperoxid, Dibenzoylperoxid, p-Chlorbenzoylperoxid, 2,4-Dictuorbenzoylperoxid, Methylethylketonperoxid, Methylisobutylketonperoxid, Cyclohexanonperoxid, Acetylacetonperoxid, Alkylester von Persäuren wie tert-Butylperpivalat, tert.-Butylperoctoat, tert.-Butylperbenzoat, tert.-Butylperisononalat, Monotert.-Butylpermaleinat, tert.-Butylperacetat, Percarbonate wie Dicyclohexyl- und Diisopropylpercarbonat, Hydroperoxide wie tert.-Butyl- oder Cumolhydroperoxid, Isophthal-monopersäure oder Acetylcyclohexan-sulfonylperoxid.

Gut geeignet sind auch Mischungen von Polymerisationsinitiatoren mit unterschiedlichen Zerfalltemperaturen.
Der Initiator wird in Mengen von 0,01 bis 3 Gew.-%, vorzugsweise 0,1 bis 1,5 Gew.-%, bezogen auf die Summe der Komponenten A, B, C und E, dem Gemisch der Komponenten A-F zugesetzt.

Die aktivierte Mischung wird in einer wäßrigen Polymerisatlösung dispergiert. Das Verhältnis von organischer Phase zu Wasserphase soll dabei in der Regel 1:1 bis 1:10, vorzugsweise 1:1,5 bis 1:4, betragen.

In der wäßrigen Dispersion kommen die bei der Suspensionspolymerisation eingesetzten Dispergierhilfsmittel zur Anwendung. Eine Auflistung geeigneter Substanzen findet sich in der Literatur, beispielsweise in Houben-Weyl, Methoden der organischen Chemie, Band XX, 1987. Gut geeignet sind beispielsweise Polyvinylpyrrolidon, Polymethacrylsäure-Na-Salz, teilverseifte Polyvinylacetate mit Verseifungsgraden von 75 bis 98 %, Copolymere von Methacrylsäure und Methacrylsäureestern niederer Alkohole und Cellulosederivate wie Carboxymethylcellulose. Besonders bevorzugt sind Copolymerisate aus Methacrylsäure und $C_1$-$C_4$-Alkylmethacrylaten in Form ihrer Na-Salze.

Die Polymerisation wird durch Erhitzen auf die Zerfallstemperatur des Polymerisationsinitiators eingeleitet. Bei einsetzender exothermer Reaktion muß gegebenenfalls gekühlt werden. Es kann vorteilhaft sein, die Polymerisation unter erhöhtem Druck, beispielsweise unter 2 bis 6 bar Stickstoffdruck durchzuführen.

Aus der auspolymerisierten Dispersion kann das Perlpolymerisat auf an sich bekannter Weise durch Dekantieren, Filtrieren, Waschen und Trocknen gewonnen werden. Das Verdünnungsmittel wird dann durch Absaugen im Vakuum vollständig entfernt.

Es ist besonders vorteilhaft, das Verdünnungsmittel vor dem Isolieren des Perlpolymerisats aus der auspolymerisierten Dispersion durch Destillation zu entfernen.

Dieses gelingt besonders gut bei Verdünnungsmitteln, die einen niedrigeren Siedepunkt als Wasser aufweisen oder mit Wasser ein Azeotrop bilden.

Die erfindungsgemäßen Perlpolymerisate weisen einen Quellungsgrad von 50 bis 2.000 Gew.-%, vorzugsweise 100 bis 1.000 Gew.-%, auf. Unter dem Quellungsgrad versteht man das Aufnahmevermögen an Flüssigkeit.

$$\text{Quellungsgrad} = \frac{\text{Gewicht der aufgenommenen Flüssigkeit}}{\text{Gewicht des Polymerisates}} \cdot 100$$

Der Quellungsgrad wird gemessen durch das Aufnahmevermögen an Tetrahydrofuran bei 20 °C.

Die erfindungsgemäßen Perlpolymerisate eignen sich zur Herstellung von polymerisierbaren Dentalmassen, insbesondere plastischen Zahnfüllungsmaterialien mit fester Konsistenz.

Ein weiterer Gegenstand der Erfindung sind polymerisierbare Dentalmassen, insbesondere plastische Zahnfüllungsmaterialien, enthaltend 20 bis 60 Gew.-%, vorzugsweise 25 bis 50 Gew.-%, monomeren Binder, 30 bis 78 Gew.-%, vorzugsweise 50 bis 70 Gew.-%, Füllstoff, 0,5 bis 30 Gew.-%, vorzugsweise 1 bis 10 Gew.-%, vernetztes Perlpolymerisat sowie gegebenenfalls an sich bekannte Zuschlagsstoffe, dadurch gekennzeichnet, daß das vernetzte Perlpolymerisat die oben angegebenen Kriterien erfüllt.

Die erfindungsgemäßen polymerisierbaren Dentalmassen können in beliebig fester Konsistenz eingestellt werden. Sie sind gut modellierbar und lassen sich nach der Stopftechnik verarbeiten, wodurch wichtige Kriterien für den Einsatz als Zahnfüllungsmaterial im Seitenzahnbereich erfüllt sind.

Die erfindungsgemäßen Dentalmassen werden durch Mischen der Ausgangskomponenten in üblichen Mischaggregaten, wie z.B. Knetern hergestellt, wobei sich die folgende Arbeitsweise als besonders vorteilhaft erweist:

Die Ausgangskomponenten werden in einem Kneter zu einer gut fließfähigen Masse, mit technisch leicht beherrschbarer Konsistenz vermischt. Diese Masse wird gegebenenfalls eingefärbt, portioniert und verpackt. Die endgültige feste Konsistenz erhält die Dentalmasse durch eine nachgeschaltete Reifung. Das Ausmäß der Nachverstrammung während der Reifung läßt sich durch den Quellungsgrad und die Einsatzmenge des erfindungsgemäßen Perlpolymerisates steuern.

Die Nachverstrammung ist nach einem bestimmten Zeitraum, der sogenannten Reifezeit, abgeschlossen. Nach dieser Zeit ist die Konsistenz über lange Zeiträume, z.B. 12 Monate konstant. Die Reifezeit dauert bei Raumtemperatur in der Regel einige Tage. Durch Temperaturerhöhung auf beispielsweise 50 °C oder 60 °C läßt sie sich auf Stunden reduzieren. Auch die Reifung bei erhöhter Temperatur führt zu einem stabilen Konsistenzniveau.

Als Füllstoffe für die erfindungsgemäßen Dentalmassen eignen sich die weiter oben als Komponente D diskutierten, rein anorganischen Füllstoffe. Ihr mittlerer Teilchendurchmesser sollte im Bereich von 0,01 bis 30 µm, vorzugsweise 0,01 bis 10 µm, besonders bevorzugt 0,02 bis 5 µm, liegen. Es werden vorzugsweise mit Haftvermittlern behandelte Füllstoffe eingesetzt. Weiterhin gut geeignete Füllstoffe sind füllstoffhaltige Perlpolymerisate gemäß DE-A-284 936 und DE-A-3 201 109, ferner mikroporöse Füllstoffe gemäß DE-A-3 430 801.

Als monomere Binder eignen sich in erster Linie Ester der Acrylsäure oder Methacrylsäure. Bevorzugt sind (Meth)acrylsäureester mit 2 oder mehr (Meth)acrylgruppen im Molekül, beispielhaft seien genannt:
Triethylenglykoldimethacrylat, Tetraethylenglykoldimethacrylat, 1,12-Dodecandioldimethacrylat, 1,6-Hexandioldimethacrylat, Diethylenglykoldimethacrylat, 2,2-Bis(p-(2'-hydroxy-3'-methacryloyloxypropoxy)phenyl)-propan, 2,2-Bis(p-(2'-methacryloyloxyethoxy)phenyl)-propan, Trimethylolpropan-tri(meth)acrylat, Bis((Meth)-acryloyloxymethyl)tricyclo[5.2.1.$0^{2,6}$]decan (gemäß DE-OS 2 931 925 und DE-OS 2 931 926), 1,3-Di((meth)-acryloyloxypropyl)-1,1,3,3-tetra-methyldisiloxan.

Ebenfalls gut geeignete monomere Binder sind Urethan(meth)acrylate gemäß den DE-OSen 3 625 202, 3 703 120, 3 636 189, 3 703 680, 3 703 130.

Es ist bevorzugt, Mischungen verschiedener (Meth)acrylsäureester einzusetzen.

Die erfindungsgemäßen Dentalmassen enthalten an sich bekannte Radikalbildner als Härtungsinitiatoren. Bevorzugt sind Härtungsinitiatoren für die Polymerisation mit Licht, beispielsweise UV, sichtbarem Licht oder Laserlicht.

Entsprechende Fotopolymerisationsinitiatoren sind bekannt, vorzugsweise handelt es sich dabei um Carbonylverbindungen, wie Benzoin und dessen Derivate, insbesondere Benzoinmethylether, Benzil und Benzilderivate, beispielsweise 4,4-Oxydibenzil oder andere Dicarbonylverbindungen, z.B. Diacetyl, 2,3-Pentandion oder Metallcarbonyle, Chinone oder deren Derivate. Der Anteil an solchen Fotopolymerisationsinitiatoren beträgt vorzugsweise etwa 0,01 bis etwa 5 Gew.-% der Gesamtzusammensetzung.

Diese mit Licht härtbaren, d.h. fotopolymerisierbaren Präparate enthalten vorzugsweise auch noch Substanzen, die in Gegenwart von Fotopolymerisationsinitiatoren die Polymerisationsreaktion beschleunigen. Bekannte Beschleuniger sind beispielsweise aromatische Amine wie p-Toluidin und Dimethyl-p-toluidin, Trialkylamine wie Trihexylamin, Polyamine, wie N,N,N',N'-Tetraalkyl-alkylendiamine, Barbitursäure und Dialkylbarbitursäuren und Sulfimide, vorzugsweise in einer Menge von etwa 0,01 bis etwa 5 Gew.-% der Gesamtzusammensetzung.

Es ist schließlich zweckmäßig, den erfindungsgemäßen Massen UV-Stabilisatoren zuzusetzen, um das Nachdunkeln während des Alterns zu vermeiden.

Ein besonders geeigneter UV-Stabilisator ist 2-Hydroxy-4-methoxybenzophenon. Ein weiteres bevorzugtes Material ist 2-(2'-Hydroxy-5'-methylphenyl)-benzotriazol; jedoch ist prinzipiell jedes physiologisch inerte UV-absorbierende Agens für diesen Zweck geeignet. So seien beispielhaft noch Hydrochinon, p-Benzochinon, p-Butylhydroxytoluol u.ä. genannt. Die letztere Verbindung kann beispielsweise auch als Antioxidans in der Füllung wirken.

Eine Übersicht über die in dentalen Füllungsmaterialien üblicherweise zum Einsatz gelangenden Substanzen findet sich in dem Artikel von R.L. Bowen im Journal of Dental Research, Vol. 58/5 (Mai 1979), S. 1493-1503, sowie der daran angeschlossenen Ergänzung von J.F. Lann, S. 1504-1506, sowie den dort zitierten Literaturstellen.

Zur Einstellung eines möglichst naturgetreuen Eindrucks der Dentalmassen enthalten sie erforderlichenfalls auch einen geringen Anteil an Farbstoffen oder Pigmenten.

Bei Anwendung großer Anteile von mehrfunktionellen Monomeren (Vernetzern) und für bestimmte Einsatzzwecke kann es vorteilhaft sein, den erfindungsgemäßen polymerisierbaren Massen Weichmacher zur Verringerung der Sprödigkeit zuzusetzen. Gut geeignet sind in erster Linie an sich bekannte hochmolekulare Weichmacher, besonders solche auf Basis von Polyurethanen, Polycarbonaten, Polyestern und Polyethern. Bevorzugt werden Polyester und Polyestercarbonate, die in DE-OS 3 316 851 beschrieben

werden.

Beispiel 1

Herstellung eines erfindungsgemäßen Perlpolymerisates

a) Monomergemisch

In einem Reaktionsgefäß, das mit einem Schnellrührer ausgerüstet ist, werden 34,5 g Methylmethacrylat, 37,5 g Isobutylmethacrylat, 18 g 2,2-Bis[p-(2'-hydroxy-3'-methacryloxypropoxy)phenyl]-propan (BisGMA) 12,6 g γ-Methacryloxypropyltrimethoxysilan, 2 g Dicyclohexylamin und 100 g n-Pentanol eingewogen und vermischt. In die erhaltene Lösung werden 210 g Bariumglasfüllstoff mit einem mittleren Teilchendurchmesser von 1,3 µm dispergiert. Die Dispersion wird 5 Stunden bei 50 °C gerührt, anschließend auf 20 °C abgekühlt und mit 1,88 g Dibenzoylperoxid und 1,88 g Dicyclohexylpercarbonat aktiviert.

b) Perlpolymerisation

In einem Reaktionsgefäß mit Ankerimpellerrührer werden 32 g Poly(methylmethacrylat -co-methacrylsäure) in 1.300 ml Wasser dispergiert. Es wird soviel 1n-NaOH zugesetzt, daß eine klare Lösung mit einem pH-Wert von 8 gebildet wird. In dieser Lösung wird das Monomergemisch emulgiert. Die Temperatur wird innerhalb von 15 Minuten auf 75 °C erhöht. Bei einsetzender exothermer Reaktion wird so stark gekühlt, daß die Temperatur im Bereich von 75 °C bis 85 °C gehalten wird. Nach dem Abklingen der Reaktion wird 2 Stunden bei 85 °C nachgerührt.

Das gebildete Perlpolymerisat wird durch Sedimentieren und Abdekantieren isoliert, gründlich mit Wasser gewaschen und bei 80 °C im Vakuum (0,1 Torr) bis zur Gewichtskonstanz getrocknet, wobei das Pentanol vollständig entfernt wird. Man erhält 260 g Perlpolymerisat mit einer mittleren Teilchengröße von 22 µm, einen Füllstoffanteil von 70 % und einem Quellungsgrad bestimmt in THF von 130 %.

Beispiel 2

Beispiel 1 wird wiederholt, wobei anstelle von Pentanol Toluol eingesetzt wird. Vor der Isolierung des Perlpolymerisates wird die Hauptmenge an Toluol destillativ entfernt. Man erhält 255 g Perlpolymerisat mit einer mittleren Teilchengröße von 28 µm, einem Füllstoffanteil von 70 % und einem Quellungsgrad von 160 %.

Beispiel 3

Nach der in Beispiel 1 angegebenen Arbeitsweise werden aktivierte Monomermischungen der folgenden Zusammensetzungen hergestellt.

| | A | B | C |
|---|---|---|---|
| Methylmethacrylat | 80 g | 80 g | 80 g |
| Isopropylmethacrylat | 76 g | 76 g | 76 g |
| Bis-GMA | 39 g | 39 g | 39 g |
| γ-Methacryloxypropyltrimethoxysilan | 6,3 g | 6,3 g | 6,3 g |
| Dicyclohexylamin | 1 g | 1 g | 1 g |
| Hochdisperses Siliciumdioxid (BET-Oberfläche 50 $m^2/g$) | 105 g | 105 g | 105 g |
| Toluol | 195 g | 244 g | 293 g |

Die aktivierten Monomerlösungen werden wie in Beispiel 1b beschrieben zu Perlpolymerisaten umgesetzt, wobei vor der Isolierung des Perlpolymerisates die Hauptmenge des Toluols destillativ entfernt wird.

Es werden Perlpolymerisate mit folgenden Kenngrößen erhalten:

| | A | B | C |
|---|---|---|---|
| Ausbeute | 261 g | 274 g | 281 g |
| mittlere Teilchengröße | 14 µm | 18 µm | 24 µm |
| Quellungsgrad in THF | 205 % | 260 % | 280 % |
| Füllstoffgehalt | 34,5 % | 34,5 % | 34,8 % |

Beispiel 4

3,0 g einer Mischung aus 70 Gew.-Teilen des Reaktionsproduktes aus 2(3),7(8)Bis(isocyanatomethyl)-tricyclo[5.2.1.$0^{2,6}$]decan mit 2-Hydroxyethylacrylat, 20 Gew.-Teilen Trimethylolpropantriacrylat und 10 Gew.-Teilen Neopentylglykoldimethacrylat werden mit 0,45 g silanisierter hochdisperser Kieselsäure (Aerosil 300 Fa. Degussa) und 6 g silanisiertem Bariumglas (GM 27884 Fa. Schott) zu einer Paste verarbeitet.

In 10 g dieser Paste werden 0,1 g bzw. 0,5 g Perlpolymerisat aus Beispiel 3 eingearbeitet.

Diese Pasten werden anschließend in einem Trockenschrank bei 45 °C gelagert und die Quellung mit einem Penetrometer verfolgt (Fa. SUR, Messung nach 30 sec, 30 Skalenteile (Sktle) ≙ 3 mm Penetration).

Die gequollenen Pasten - vor allem die mit 5 % Perlpolymerisat - zeigen das für ein Seitenzahncomposit gewünschte stopfbare, klebfreie Verarbeitungsverhalten.

| Lagerzeit | 1 % Perlpolymerisat Beispiel 3 A | 1 % Perlpolymerisat Beispiel 3 B | 1 % Perlpolymerisat Beispiel 3 C | 5 % Perlpolymerisat Beispiel 3 A | 5 % Perlpolymerisat Beispiel 3 B | 5 % Perlpolymerisat Beispiel 3 C |
|---|---|---|---|---|---|---|
| Ausgangswert | 29,3 Sktle | 29,3 Sktle | 29,5 Sktle | 29,4 Sktle | 29,3 Sktle | 29,5 Sktle |
| 18 Stunden 45°C | 29,0 Sktle | 28,6 Sktle | 28,6 Sktle | 29,1 Sktle | 27,1 Sktle | 18,1 Sktle |
| 6 Tage 45°C | 27,1 Sktle | 27,1 Sktle | 27,1 Sktle | 25,8 Sktle | 15,4 Sktle | 10,7 Sktle |
| 11 Tage 45°C | 27,7 Sktle | 26,9 Sktle | 26,4 Sktle | 25,2 Sktle | 16,1 Sktle | 11,6 Sktle |

## Patentansprüche

1. Vernetzte Perlpolymerisate, dadurch gekennzeichnet, daß sie einen mittleren Teilchendurchmesser von 2 bis 100 µm, einen Gehalt an anorganischem Füllstoff von 10 bis 75 Gew.-% und einen Quellungsgrad

von 50 bis 2.000 % gemessen in THF aufweisen.

2. Vernetzte Perlpolymerisate nach Anspruch 1, dadurch gekennzeichnet, daß sie aus
   A: 10 bis 78 Gew.-% $C_1$-$C_2$-Alkyl-(meth)acrylat
   B: 5 bis 50 Gew.-% $C_3$-$C_{12}$-Alkyl-(meth)acrylat
   C: 1 bis 30 Gew.-% Vernetzer
   D: 10 bis 75 Gew.-% anorganischer Füllstoff
   und gegebenenfalls
   E: 0 bis 30 Gew.-% weiteren ethylenisch ungesättigten Monomeren
   aufgebaut sind.

3. Verfahren zur Herstellung von Perlpolymerisaten nach Anspruch 1, dadurch gekennzeichnet, daß man eine Mischung aus
   A: 10 bis 78 Gew.-Teilen $C_1$-$C_2$-Alkyl-(meth)acrylat
   B: 5 bis 50 Gew.-Teilen $C_3$-$C_{12}$-Alkyl-(meth)acrylat
   C: 1 bis 30 Gew.-Teilen Vernetzer
   D: 10 bis 75 Gew.-Teilen anorganischen Füllstoff
   gegebenenfalls
   E: 0 bis 30 Gew.-Teilen weiteren ethylenisch ungesättigten Monomeren
   und
   F: 50 bis 1.000 Gew.-Teilen nichtwäßriges Verdünnungsmittel
   in einer wäßrigen Polymerisatlösung dispergiert und unter Anwendung eines Initiators nach der Verfahrensweise der Suspensionspolymerisation polymerisiert.

4. Verwendung von vernetzten Perlpolymerisaten gemäß Anspruch 1 in polymerisierbaren Dentalmassen.

5. Verwendung von vernetzten Perlpolymerisaten gemäß Anspruch 1 in polymerisierbaren Dentalmassen, enthaltend 20 bis 60 Gew.-% monomeren Binder, 30 bis 78 Gew.-% Füllstoff und 0,5 bis 30 Gew.-% des vernetzten Perlpolymerisats sowie gegebenenfalls an sich bekannte Zuschlagstoffe.

6. Verwendung von vernetzten Perlpolymerisaten gemäß Anspruch 1 in polymerisierbaren Dentalmassen enthaltend 25 bis 50 Gew.-% monomeren Binder, 50 bis 70 Gew.-% Füllstoff und 0,5 bis 30 Gew.-% des vernetzten Perlpolymerisats sowie gegebenenfalls an sich bekannte Zuschlagstoffe.

## Claims

1. Cross-linked bead polymers, characterized in that they have an average particle diameter of 2 to 100 µm, a content of inorganic filler of 10 to 75% by weight and a degree of swelling of 50 to 2000%, measured in THF.

2. Cross-linked bead polymers according to Claim 1, characterized in that they are synthesized from
   A: 10 to 78% by weight of $C_1$-$C_2$-alkyl (meth)acrylate
   B: 5 to 50% by weight of $C_3$-$C_{12}$-alkyl (meth)acrylate
   C: 1 to 30% by weight of cross-linker
   D: 10 to 75% by weight of inorganic fillers
   and, if desired,
   E: 0 to 30% by weight of other ethylenically unsaturated monomers.

3. Process for the preparation of bead polymers according to Claim 1, characterized in that a mixture of
   A: 10 to 78 parts by weight of $C_1$-$C_2$-alkyl (meth)acrylate
   B: 5 to 50 parts by weight of $C_3$-$C_{12}$-alkyl (meth)acrylate
   C: 1 to 30 parts by weight of cross-linker
   D: 10 to 75 parts by weight of inorganic filler
   if desired
   E: 0 to 30 parts by weight of other ethylenically unsaturated monomers
   and
   F: 50 to 1000 parts by weight of non-aqueous diluent
   is dispersed in an aqueous polymer solution and polymerized by the suspension polymerization

process using an initiator.

4. Use of cross-linked bead polymers according to Claim 1 in polymerizable dental materials.

5. Use of cross-linked bead polymers according to Claim 1 in polymerizable dental materials containing 20 to 60% by weight of monomeric binder, 30 to 78% by weight of filler and 0.5 to 30% by weight of the cross-linked bead polymer and, if desired, additives known per se.

6. Use of cross-linked bead polymers according to Claim 1 in polymerizable dental materials containing 25 to 50% by weight of monomeric binder, 50 to 70% by weight of filler and 0.5 to 30% by weight of the cross-linked bead polymer and, if desired, additives known per se.

**Revendications**

1. Polymères réticulés en perles, caractérisés en ce qu'ils ont un diamètre moyen de particules de 2 à 100 µm, une teneur en charge inorganique de 10 à 75 % en poids et un degré de gonflement de 50 à 2000 %, mesuré dans le THF.

2. Polymères réticulés en perles suivant la revendication 1, caractérisé ce qu'ils sont constitués
   A : de 10 à 78 % en poids de (méth)acrylate d'alkyle en $C_1$ ou $C_2$
   B : de 5 à 50 % en poids de (méth)acrylate d'alkyle en $C_3$ à $C_{12}$
   C : de 1 à 30 % en poids d'un agent réticulant
   D : de 10 à 75 % en poids de charge inorganique
   et le cas échéant
   E : de 0 à 30 % en poids d'autres monomères à non-saturation éthylénique

3. Procédé de production de polymères en perles suivant la revendication 1, caractérisé en ce qu'on disperse dans une solution aqueuse de produit de polymérisation, un mélange constitué
   A : de 10 à 78 parties en poids de (méth)acrylate d'alkyle en $C_1$ ou $C_2$
   B : de 5 à 50 parties en poids de (méth)acrylate d'alkyle en $C_3$ à $C_{12}$
   C : de 1 à 30 parties en poids d'un agent réticulant
   D : de 10 à 75 parties en poids d'une charge inorganique
   le cas échéant
   E : de 0 à 30 parties en poids d'autres monomères à non-saturation éthylénique
   et
   F : de 50 à 1000 parties en poids de diluant non aqueux
   et on conduit la polymérisation en utilisant un initiateur selon le principe opératoire de la polymérisation en suspension.

4. Utilisation de polymères réticulés en perles suivant la revendication 1, dans des compositions dentaires polymérisables.

5. Utilisation de polymères réticulés en perles suivant la revendication 1, dans des compositions dentaires polymérisables, contenant 20 à 60 % en poids de liant monomère, 30 à 78 % en poids de charge et 0,5 à 30 % en poids du polymère réticulé en perles ainsi que, le cas échéant, des additifs connus.

6. Utilisation de polymères réticulés en perles suivant la revendication 1, dans des compositions dentaires polymérisables contenant 25 à 50 % en poids de liant monomère, 50 à 70 % en poids de charge et 0,5 à 30 % en poids du polymère réticulé en perles ainsi que, le cas échéant, des additifs connus.